# DEMANDE DE BREVET EUROPEEN

(11) **EP 1 787 952 A1**
(43) Date de publication de la demande: **23.05.2007**
(21) Numéro de dépôt: 05300937.9
(22) Date de dépôt: 17.11.2005
(51) Int. Cl.: C01B 6/21, C01B 3/06, C07F 5/04, C01B 35/12, C01B 6/10, C07C 29/68, C07C 29/132

(54) **Procédé de préparation d'alcoolate alcalin et sa mise en oeuvre pour régénérer du borohydrure de sodium à partir de métaborate de sodium**

(71) Demandeur: L'AIR LIQUIDE, Société Anonyme pour l'Etude et l'Exploitation des Procédés Georges Claude, 75321 Paris Cedex 07 (FR)
(72) Inventeur: Minkina, Valentina, Gamarnika 24-92 220131 Minsk (BY); Barral, Katia, 78150, LE CHESNAY (FR)
(74) Mandataire: Conan, Philippe Claude

(57) **Abrégé**

Procédé de préparation d'un alcoolate de formule (IV) :

ZOR (IV),

dans laquelle Z représente un cation monovalent dérivé d'un métal alcalin et R représente un radical alkyle linéaire ou ramifié comportant de un à quatre atomes de carbone, d'un borate de trialkyle de formule (V) :

B(OR)₃ (V)

dans laquelle R est tel que défini dans la formule (IV) ci-dessus, et d'eau,
caractérisé en ce qu'il comprend :
une étape (a₁) de réaction d'un métaborate de formule (II) :

Z(BO₂) (II)

dans laquelle Z est tel que défini dans la formule (IV) ci-dessus, avec un alcool de formule (III) :

ROH (III)

dans laquelle R est tel que défini dans la formule (IV) ci-dessus, pour former un composé de formule (VII) :

Z[B(OR)₄] (VII)

selon l'équation chimique :

ZBO₂ + 4ROH → Z[B(OR)_{4]} + 2 H₂O ;

une étape (a₂) de clivage du composé de formule (VII) en composé de formule (IV) et en composé de formule (V) telles que définies ci-dessus. selon l'équation chimique

Z[B(OR)₄] → ZOR + B(OR)₃ ;

procédé de régénération de borohydrure de sodium, à partir du métaborate de sodium.

## Description

L'invention a pour objet un nouveau procédé de préparation de borohydrure de sodium.

L'hydrogène est un carburant "dit propre" alternatif aux carburants fossiles, en ce que sa combustion conduit uniquement à la formation d'eau. Cependant l'hydrogène n'est pas facile à stocker ce gaz diffuse aisément à travers un grand nombre de matériaux. C'est pour cette raison que l'on envisage pour certaines applications industrielles un stockage dit chimique de ce gaz. On entend par stockage chimique, le stockage d'un composé susceptible de libérer de l'hydrogène par une réaction chimique simple, ce gaz étant utilisé ensuite sans nouveau stockage. Parmi les composés chimiques candidats, il y a les hydrures et plus particulièrement les borohydrures, tels que le borohydrure de sodium NaBH₄. Ce composé libère de l'hydrogène par une simple réaction dans l'eau répondant à l'équation chimique:

NaBH₄ + 2 H₂O → NaBO₂ + 4 H₂

Les avantages d'un tel mode de stockage sont nombreux:
Seul n'est produit comme gaz, que l'hydrogène nécessaire à la mise en oeuvre de l'application ;
L'hydrogène produit est à basse pression ; le stockage du borohydrure de sodium est aussi un stockage basse pression (pression ambiante sous forme liquide, solide ou pâte) et à température ambiante ; le stockage donc plus sûr.

En cours d'utilisation il y a peu d'hydrogène dans l'installation, sauf dans les canalisations dédiées.

Cependant le borohydrure de sodium est produit à partir de borate de sodium et les procédés actuels conduisent à un produit relativement cher qui est essentiellement utilisé en chimie fine comme agent réducteur. Dans une utilisation en tant que stockage d'hydrogène, le borohydrure conduit à un coût de l'hydrogène produit et/ou stocké non compétitif comparé à celui produit et/ou stocké par les méthodes classiques comme les stockages conventionnels haute pression ou liquide. De plus en cas de consommation à grande échelle (utilisation pour des applications énergétiques par exemple), le métaborate de sodium NaBO₂, sous-produit de la réaction susmentionnée doit être recyclé à un coût compétitif.

Parmi les procédés connus de transformation du NaBO₂ en NaBH₄, il y a ceux décrits :
(i) Dans le brevet américain US 6,524,542 qui se déroulent selon les étapes suivantes :

   4 YBO₂ + 4 CO₂ + 2 H₂O → 2 B₂O₃ + 4 YHCO₃ (Y représentant un cation monovalent),

   4 YHCO₃ → 2 Y₂O + 2 H₂O + 4 CO₂

   2 B₂O₃ + 6 C + 6 X₂ → 4 BX₃ + 6 CO (X représentant un atome de chlore, de brome, d'iode ou de fluor),

   4BX₃ + 12H₂ → 2B₂H₆+12 HX

   2 Y₂O + 2 B₂H₆ → 3 YBH₄ + YBO₂

   et

   4 YBO₂ + 4 CO₂ + 2 H₂O → 2 B₂O₃ + 4 YHCO₃ (Y représentant un cation monovalent),

   4 YHCO₃ → 2 Y₂CO₃ + CO₂ + 2 H₂O

   2 Y₂CO₃ + 2 B₂H₆ → 3 YBH₄ + Y BO₂ + 2 CO₂

   X représentant de préférence un atome de brome et Y représente un atome de sodium ;
   (ii) Dans le brevet américain US 6,586,563 qui divulgue des réactions suivantes :

      2 BH₃(NEt₃) + 2 NaOH → NaBH₄ + NaBO₂ + 2 H₂ + 2 NEt₃,

      4 BH₃(NEt₃) + 2 Na₂CO₃ → 3 NaBH₄ + NaBO₂ + 2 CO₂+ 2 NEt₃,

      B₂H₆ + 2 NaOH → NaBH₄ + NaBO₂ + 2 H₂,

      et

      2 B₂H₆ + 2 Na₂CO₃ → 3 NaBH₄ + NaBO₂ + 2 CO₂.

      ces réactions se déroulant à des températures proches de l'ambiante (-5°C / +20°C) et sous pression atmosphérique (atmosphère inerte).
   (iii) Dans la demande internationale WO 02/062701 qui divulgue les séquences suivantes :

      NaBO₂ + MgH₂ → NaBH₄ + 2 MgO,

      et

      NaBO₂ + 2 Mg + Si + 2 H₂ → NaBO₂ + 2 Mg₂Si + 2 H₂

      NaBO₂ + 2 Mg₂Si + 2 H₂ → NaBH₄ + 2 MgO + Si.

      ces réactions se déroulant à des températures élevées (550 °C) et sous atmosphère d'hydrogène à une pression de 70.10⁵ Pa (70 bars). Bien que ce dernier procédé, contrairement aux précédents, ne produise pas de dioxyde de carbone, ses conditions réactionnelles sont difficiles à mettre en oeuvre.

C'est pourquoi les inventeurs se sont attachés à développer un nouveau procédé mettant en oeuvre des oxydes de bore et des dérivés de cation alcalin qui ne produise pas de dioxyde de carbone, qui puisse conduire à un cycle clos en éléments alcalins et bore, qui ne pas consomme de gaz naturel, et qui puisse être mis en oeuvre sous des conditions opératoires plus douces que celles des procédés de l'état de la technique.

L'invention a donc pour objet un procédé de préparation d'un alcoolate de formule (IV) :

ZOR (IV),

dans laquelle Z représente un cation monovalent dérivé d'un métal alcalin et R représente un radical alkyle linéaire ou ramifié comportant de un à quatre atomes de carbone, d'un borate de trialkyle de formule (V):

B(OR)₃ (V)

dans laquelle R est tel que défini dans la formule (IV) ci-dessus, et d'eau,
caractérisé en ce qu'il comprend :
une étape (a₁) de réaction d'un métaborate de formule (II):

   Z(BO₂) (II)

   dans laquelle Z est tel que défini dans la formule (IV) ci-dessus, avec un alcool de formule (III):

   ROH (III)

   dans laquelle R est tel que défini dans la formule (IV) ci-dessus, pour former un composé de formule (VII):

   Z[B(OR)₄] (VII)

   selon l'équation chimique :

   ZBO₂ + 4ROH → Z[B(OR)_{4]} + 2 H₂O;

   et
une étape (a₂) de clivage du composé de formule (VII) en composé de formule (IV) et en composé de formule (V) telles que définies ci-dessus.
selon l'équation chimique

Z[B(OR)₄] → ZOR + B(OR)₃

Dans le procédé tel que défmi ci-dessus, par cation monovalent dérivé d'un métal alcalin, on désigne pour Z, notamment le cation lithium, le cation sodium ou le cation, potassium et par radical alkyle linéaire ou ramifié comportant de un à quatre atomes de carbone, on désigne pour R notamment un radical choisi parmi les radicaux méthyle, éthyle, propyle, isopropyle, butyle, isobutyle, sec-buyle ou ter-butyle.

Selon un autre aspect du procédé tel que défini ci-dessus, les étapes a₁ et a₂: sont réalisées au sein d'une seule étape (a), selon l'équation chimique:

Z(BO₂) + 4 ROH → ZOR + B(OR)₃ + 2 H₂O.

Selon un autre aspect du procédé tel que défini ci-dessus, celui-ci comprend en outre étape (b) de réduction du borate de trialkyle de formule (V) en diborane de formule (VIII) :

B₂H₆ (VIII)

et en alcool de formule (III), selon l'équation chimique:

2B(OR)₃ + 6H₂ → B₂H₆ + 6 ROH

Selon ce dernier aspect le procédé peut aussi comprendre une étape (c) de réaction du diborane de formule (VIII) avec l'alcoolate de formule (IV) pour former le borohydrure de formule (I):

ZBH₄ (I)

et le borate de trialkyle de formule (V), selon l'équation chimique:

3 ZOR + 2B₂H₆ → 3ZBH₄ + B(OR)₃

Dans le procédé tel que défmi précédemment, dans les formules (III), (IV) et (V), R représente par exemple un radical méthyle.

Selon un autre du procédé tel que défini précédemment, dans les formules (I), (II), (IV), (VI) et (VII), Z représente un cation sodium.

L'invention a aussi pour objet, un procédé de régénération de borohydrure de sodium, à partir du métaborate de sodium produit simultanément à l'hydrogène issu d'un stockage sous forme de borohydrure de sodium, par hydrolyse dudit borohydrure de sodium selon l'équation chimique suivante :

NaBH₄ + 2 H₂O → NaBO₂ + 4 H₂,

caractérisé en ce qu'il met en oeuvre le procédé tel que défini précédemment qui comprend les étapes successives a1, a2, b et c ou les étapes successives a, b et c, telles que définies ci-dessus.

Une telle régénération est avantageuse dans le cas d'une installation industrielle qui serait grosse consommatrice d'hydrogène donc grosse productrice de métaborate de sodium. Elle s'effectue globalement selon l'équation chimique suivante:

NaBO₂ + 3H₂ → NaBH₄ + H₂O

Les expériences décrites dans le paragraphe suivant illustrent l'invention sans toutefois la limiter.

### 1- Préparation de tétraméthoxy borate de sodium NaB(OCH₃)₄

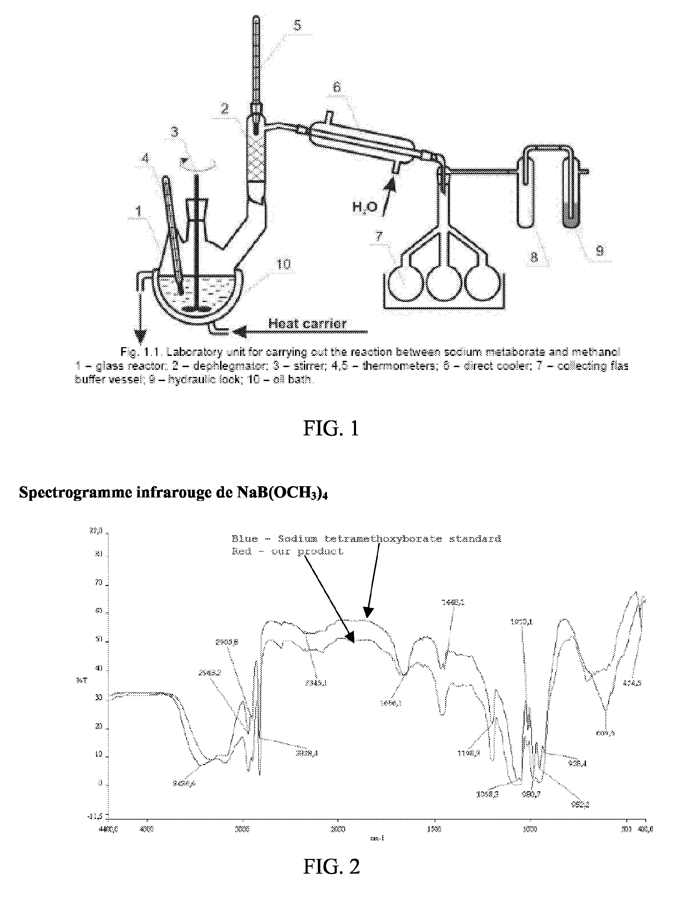

Ce composé est préparé en utilisant le montage illustré par la figure 1. On prépare dans le ballon, une suspension de métaborate de sodium dans un excès de méthanol dans la proportion molaire NaBO₂ / CH₃OH = 1,8. Le ballon est chauffé au reflux à 65-68°C pendant 30 à 60 minutes environ, en distillant l'eau produite et en rajoutant huit fois dans le ballon, du méthanol pour compenser la perte induite par la distillation de l'eau. L'analyse en eau des 8 distillats dont les résultats sont consignés dans le tableau suivant, traduit l'avancement de la réaction et la consommation du réactif métaborate de sodium.

| ajout | 1 | 2 | 3 | 4 | 5 | 6 | 7 | 8 |
|---|---|---|---|---|---|---|---|---|
| % H₂O | 15,43 | 18,29 | 14,37 | 12,06 | 6,44 | 7,69 | 3,91 | 2,10 |

Le solide obtenu après ces 8 ajouts de méthanol est séché au rotovapor.
Le NaB(OCH₃)₄ est obtenu avec un rendement de 74,17%.
Le point de fusion du produit final est de 176-180°C. La Figure 2 représente les spectres infrarouges du produit du produit final (spectrogramme rouge) par rapport à celui d'un échantillon standard de NaB(OCH₃)₄ (spectrogramme bleu)

### 2 - Préparation de borate de triméthyle

### [B(OCH₃)₃]

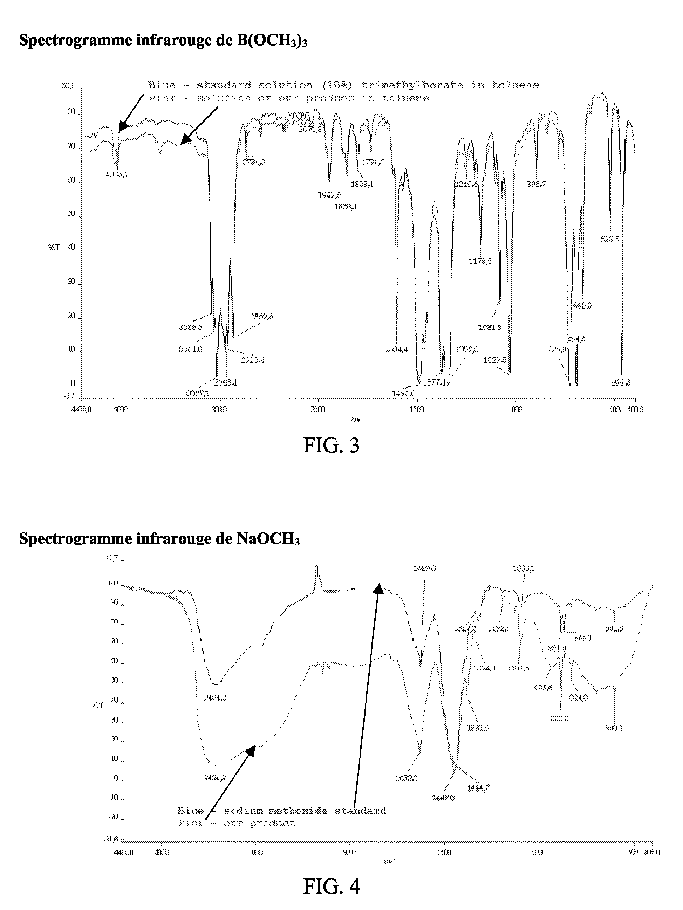

Le métaborate de sodium produit lors de l'étape 1 est placé dans un ballon et chauffé dans un bain d'huile à 280°C sous vide pendant 60 à 100 min. Les vapeurs sont condensées dans un piège refroidi à l'azote liquide 77 K. Le borate de triméthylet [B(OCH₃)₃] ainsi récupéré est dilué dans du toluène pour analyse. La Figure 3 représente les spectres infrarouges du B(OCH₃)₃ récupéré (spectrogramme rouge) par rapport à celui d'un échantillon standard de B(OCH₃)₃ (spectrogramme bleu). Le résidu contenu le ballon à la fin du temps imparti est un mélange de méthoxylate de sodium (NaOCH₃). La Figure 4 représente les spectres infrarouges du NaOCH₃ récupéré (spectrogramme rouge) par rapport à celui d'un échantillon standard de NaOCH₃ (spectrogramme bleu). Le rendement de la réaction, dans les conditions citées, est de 70,12 %.

### 3 - Préparation de diborane (B₂H₆)

On met en oeuvre le procédé décrit dans le brevet US 6,433,129 B1, colonne 8, lignes 15 à 29 à partir du borate de triméthyle préparé à l'étape précédente et l'on obtient le diborane attendu et du méthanol.

### 4 - Préparation du borohydrure de sodium (NaBH₄):

On met en oeuvre le procédé décrit par H.I. Schlesinger et al ; (H.I. Schlesinger, al H.C. Brown, H.R. Hoekstra and L.R.Rapp ; J Amer Chem Soc 75 (1953) p. 199) à partir du diborane obtenu à l'étape précédente pour obtenir le borohydrure de sodium attendu et le borate de triméthyle.

## Revendications

1. Procédé de préparation d'un alcoolate de formule (IV):
ZOR (IV),
dans laquelle Z représente un cation monovalent dérivé d'un métal alcalin et R représente un radical alkyle linéaire ou ramifié comportant de un à quatre atomes de carbone, d'un borate de trialkyle de formule (V):
B(OR)₃ (V)
dans laquelle R est tel que défini dans la formule (IV) ci-dessus, et d'eau,
**caractérisé en ce qu'**il comprend :
une étape (a₁) de réaction d'un métaborate de formule (II):
Z(BO₂) (II)
dans laquelle Z est tel que défini dans la formule (IV) ci-dessus, avec un alcool de formule (III):
ROH (III)
dans laquelle R est tel que défini dans la formule (IV) ci-dessus, pour former un composé de formule (VII):
Z[B(OR)₄] (VII)
selon l'équation chimique :
ZBO₂ + 4ROH → Z[B(OR)₄] + 2 H₂O;
une étape (a₂) de clivage du composé de formule (VII) en composé de formule (IV) et en composé de formule (V) telles que définies ci-dessus.
selon l'équation chimique
Z[B(OR)₄] → ZOR + B(OR)₃

2. Procédé de préparation des composés de formules (IV) et (V) et d'eau, tel que défmi à la revendication 1, dans lequel les étapes a₁ et a₂: sont réalisées au sein d'une seule étape a, selon l'équation chimique :
Z(BO₂) + 4 ROH → ZOR + B(OR)₃ + 2 H₂O.

3. Procédé tel que défini à l'une des revendications 1 ou 2 **caractérisé en ce qu'**il comprend en outre une étape (b) de réduction du borate de trialkyle de formule (V) en diborane de formule (VIII) :
B₂H₆ (VIII)
et en alcool de formule (III), selon l'équation chimique:
2B(OR)₃ + 6H₂ → B₂H₆ + 6 ROH

4. Procédé tel que défini à la revendication 3, comprenant en outre une étape (c) de réaction du diborane de formule (VIII) avec l'alcoolate de formule (IV) pour former le borohydrure de formule (I):
ZBH₄ (I)
et le borate de trialkyle de formule (V), selon l'équation chimique:
3 ZOR + 2B₂H₆ → 3ZBH₄ + B(OR)₃

5. Procédé tel que défini à l'une des revendications 1 à 4, pour lequel, dans les formules (III), (IV) et (V), R représente un radical méthyle.

6. Procédé tel que défini à l'une des revendications 1 à 4, pour lequel, dans les formules (I), (II), (IV), (VI) et (VII), Z représente un cation sodium.

7. Procédé de régénération de borohydrure de sodium, à partir du métaborate de sodium produit simultanément à l'hydrogène issu d'un stockage sous forme de borohydrure de sodium, par hydrolyse selon l'équation chimique :
NaBH₄ + 2 H₂O → NaBO₂ + 4 H₂,
**caractérisé en ce qu'**il met en oeuvre le procédé tel que défini aux revendications 4, 5 et 6.
